# EUROPÄISCHE PATENTSCHRIFT

(11) **EP 0 712 840 B1**
(45) Veröffentlichungstag und Bekanntmachung des Hinweises auf die Patenterteilung: **03.03.1999**
(21) Anmeldenummer: 95117435.8
(22) Anmeldetag: 06.11.1995
(51) Int. Cl.: C07C 275/60, C08G 18/78

(54) **Allophanatgruppen aufweisende Polyisocyanate**
Allophanate-groups containing polyisocyanates
Polyisocyanates contenant des groupes allophanates

(30) Priorität: 18.11.1994 DE 4441176
(43) Veröffentlichungstag der Anmeldung: 22.05.1996
(73) Patentinhaber: BAYER AG, 51368 Leverkusen (DE)
(72) Erfinder: Brahm, Martin, Dr., D-51766 Engelskirchen (DE); Schmalstieg, Lutz, Dr., D-50676 Köln (DE); Pedain, Josef, Dr., D-51061 Köln (DE)

(56) Entgegenhaltungen:
- EP-A- 0 362 654
- US-A- 5 319 054

## Beschreibung

Die Erfindung betrifft ein neues Verfahren zur Herstellung von Allophanatgruppen aufweisenden Polyisocyanaten, die nach diesem Verfahren erhältlichen Verbindungen und ihre Verwendung als Bindemittel oder Bindemittelkomponente in Beschichtungsmitteln.

Allophanatgruppen enthaltende Polyisocyanate und ihre Verwendung in Beschichtungsmitteln sind seit langem bekannt (vgl. z.B. GB-PS 994 890, EP-A-0 000 194 oder EP-A-0 303 150). Die Herstellung derartiger Allophanatgruppen aufweisender Polyisocyanate erfolgt im allgemeinen durch Reaktion überschüssiger Mengen an zumeist aliphatischen oder cycloaliphatischen Diisocyanaten mit ein- oder mehrwertigen Alkoholen. Nach der Urethanisierungs- und Allophanatisierungsreaktion wird der Überschuß an flüchtigen Isocyanatverbindungen destillativ entfernt und es entstehen Produkte, deren Eigenschaftsprofil je nach eingesetztem Diisocyanat breit variiert werden kann. Die Herstellung von Allophanatgruppen aufweisenden Polyisocyanaten auf Basis von aromatischen Diisocyanaten wird beispielsweise in US-PS 3 769 318 beschrieben. Einer der Nachteile dieser aromatischen Allophanatpolyisocyanate ist in ihrer schlechten Thermostabilität zu sehen, so daß die Umsetzungsprodukte unter den Bedingungen der Dünnschichtdestillation die eingebauten Diisocyanate abspalten, was eine vollständige Abtrennung des überschüssigen Diisocyanats im Anschluß an die Allophanatisierungsreaktion unmöglich macht.

Wie jetzt gefunden wurde, ist es nach dem nachstehend näher beschriebenen erfindungsgemäßen Verfahren unter Verwendung von unterschiedlichen Isocyanatkomponenten für die Urethanisierung und die anschließende Allophanatisierung möglich, thermostabile, Allophanatgruppen aufweisende Polyisocyanate herzustellen, die unterschiedliche Isocyanatgruppen in chemisch eingebauter Form enthalten und somit nachstehend als "Heteroallophanate" bezeichnet werden. Die Eigenschaften dieser Heteroallophanate, insbesondere ihre Viskosität und Reakivität und die Eigenschaften der aus ihnen hergestellten Beschichtungen, wie beispielsweise Härte und Elastizität, können auf einfache Weise durch geeignete Wahl der zur Urethanisierung einerseits und zur Allophanatisierung andererseits eingesetzten Isocyanatkomponente gesteuert werden. Von besonderer Bedeutung ist die Thermostabilität der nachstehend näher beschriebenen erfindungsgemäßen Verfahrensprodukte auch bei Verwendung von aromatischen Diisocyanaten, insbesondere von Diisocyanatotoluol bei der Allophanatisierungsreaktion.

Der naheliegende Gedanke, die genannte Variabilität der Produkt- und Filmeigenschaften durch Verwendung von Isocyanatgemischen, insbesondere Diisocyanatgemischen bei der Urethanisierung und anschließenden Allophanatisierung zu erreichen, scheitert zum einen an der oftmals unzureichenden Thermostabilität der so erhaltenen "Mischallophanate" und ist zum anderen mit dem Nachteil behaftet, daß es sich bei den entsprechenden Umsetzungsprodukten im Gegensatz zu den nachstehend näher beschriebenen erfindungsgemäßen Verfahrensprodukten um Polyisocyanate mit Isocyanatgruppen unterschiedlicher Reaktivität handelt.

Gegenstand der Erfindung ist ein Verfahren zur Herstellung von Allophanatgruppen aufweisenden Polyisocyanaten durch Umsetzung unter Allophanatbildung von Urethangruppen aufweisenden Verbindungen a) mit bezüglich der Urethangruppen überschüssigen Mengen an destillierbaren organischen Polyisocyanaten b) und anschließende destillative Entfernung des nicht umgesetzten Überschusses der Komponente b) bis auf einen Restgehalt von unter 0,5 Gew.-%, und wobei die

Komponenten a) im wesentlichen Hydroxyl- und Isocyanatgruppen-freie, pro Molekül im statistischen Mittel mindestens zwei Urethangruppen aufweisende, durch Umsetzung von organischen Isocyanaten a1) mit organischen Hydroxylverbindungen a2) hergestellte Verbindungen eines maximalen durchschnittlichen Molekulargewichts von 2.500 sind dadurch gekennzeichnet, daß man als Komponente b) von der Komponente a1) verschiedene Polyisocyanate verwendet wobei 2,2'-, 2,4'- und 4,4'-Diisocyanatodiphenylmethan ausgenommen sind.

Ausgangsmaterialien beim erfindungsgemäßen Verfahren sind a) Urethangruppen aufweisende Verbindungen der nachstehend genauer beschriebenen Art und b) organische Polyisocyanate, die mit den Urethangruppen aufweisenden Verbindungen a) unter Allophanatisierung in an sich bekannter Weise zur Umsetzung gebracht werden.

Die Urethangruppen aufweisenden Verbindungen a) weisen (im statistischen Mittel) pro Molekül mindestens zwei Urethangruppen und ein aus der Stöchiometrie der Ausgangsmaterialien errechenbares Molekulargewicht von maximal 2.500, vorzugsweise von maximal 1.500 auf. Die Verbindungen a) sind "im wesentlichen" Isocyanat- und Hydroxylgruppen-frei. Dies soll bedeuten, daß der NCO-Gehalt der Verbindungen a) bei max. 2, vorzugsweise bei max. 0,5 und insbesondere bei max. 0,2 Gew.-% und der Hydroxylgruppengehalt bei max. 1, vorzugsweise bei max. 0,3 und insbesondere bei max. 0,1 Gew.-% liegt. Dies wird im wesentlichen dadurch erreicht, daß bei der Herstellung der Verbindungen a) die Ausgangskomponenten a1) und a2) unter Einhaltung eines NCO/OH-Äquivalentverhältnisses von 1,2:1 bis 1:1,2, vorzugsweise 1,1:1 bis 1:1,1 und besonders bevorzugt von 1:1 zur Umsetzung gebracht werden. Diese Urethanbildungsreaktion wird im allgemeinen innerhalb des Temperaturbereich- von 20 bis 130, vorzugsweise 50 bis 90°C, vorzugsweise in Substanz durchgeführt.

Die Isocyanatkomponente a1) besteht aus beliebigen (cyclo)aliphatischen und/oder aromatischen Mono-, Di- und/oder Polyisocyanaten des Molekulargewichtsbereichs 99 bis 1.000, vorzugsweise 140 bis 300 mit einem NCO-Gehalt von 10 bis 56, vorzugsweise 18 bis 56 und insbesondere 30 bis 50 Gew.-%. Die (mittlere) NCO-Funktionalität der Komponente a1) liegt bei 1 bis 6, vorzugsweise bei 2 bis 3,5 und besonders bevorzugt bei 2. Vorzugsweise besteht die Isocyanatkomponente a1) ausschließlich aus Diisocyanaten, insbesondere linearaliphatischen oder cyclischen Diisocyanaten. Unter "cyclischen" Diisocyanaten sind solche zu verstehen, die pro Molekül mindestens einen aromatischen oder mindestens einen cycloaliphatischen Ring aufweisen.

Beispiele für Monoisocyanate, die als Komponente a1) oder als Teil der Komponente a1) Verwendung finden können, sind Phenylisocyanat und (cyclo)aliphatische Monoisocyanate mit 4 bis 18 Kohlenstoffatomen wie beispielsweise n-Butylisocyanat, Cyclohexylisocyanat oder n-Stearylisocyanat.

Beispiele geeigneter Diisocyanate sind Tetramethylendiisocyanat, Pentamethylendiisocyanat, Hexamethylendiisocyanat (HDI), Undecamethylendiisocyanat, Dodecamethylendiisocyanat, 1-Isocyanato-3,3,5-trimethyl-5-isocyanatomethyl-cyclohexan (IPDI), 4,4'-Bis-(isocyanatocyclohexyl)-methan und dessen Gemischen mit den entsprechenden : 2,4'- und gegebenenfalls 2,2'-Isomeren (HMDI), 1-Methyl-2,4-diisocyanatocyclohexan oder dessen Gemische mit 1-Methyl-2-6-diisocyanatohexan (HTDI), 1-Isocyanato-4(3)-isocyanatomethyl-1-methylcyclohexan oder p-Xylylendiisocyanat sowie deren Gemische. Auch aromatische Diisocyanate wie beispielsweise 2,4- und/oder 2,6-Diisocyanatotoluol (TDI) kommen als Komponente a1) oder als Teil dieser Komponente in Betracht.

Auch Biuret-, Uretdion-, Isocyanurat- oder Carbodiimidgruppen aufweisende Derivate der oben genannten Isocyanate kommen im Prinzip als Komponente a1) bzw. als Teil dieser Komponente in Betracht, jedoch ist die (Mit)Verwendung derartiger Modifizierungsprodukte keinesfalls bevorzugt.

Die Komponente a1) besteht vorzugsweise aus HDI, HMDI, IPDI oder HTDI.

Die Alkoholkomponente a2) besteht aus 1- bis 6-wertigen Alkoholen des Molekulargewichtsbereichs 32 bis 900, vorzugsweise 74 bis 300 oder aus beliebigen Gemischen derartiger Alkohole. In Betracht kommende Alkohole sind beispielsweise gesättigte einwertige Alkohole wie z.B. Methanol, Ethanol, n-Propanol, Isopropanol, Methoxypropanol, die isomeren Butanole, Pentanole, Hexanole, Octanole, Decanole, Dodecanole, Octadecanole; mehrwertige Alkohole wie beispielsweise Ethylenglykol, Propylenglykol, Butandiol-1,4, Hexandiol-1,6, Neopentylglykol, 2-Methyl-propandiol-1,3, 2,2,4-Trimethylpentandiol-1,3, Dimerfettalkohole, Trimerfettalkohole, Glycerin, Trimethylolpropan, Trimethylolethan, die isomeren Hexantriole, Pentaerythrit oder Sorbit. Als Komponente bzw. Teil der Komponente a2) ebenfalls einsetzbar sind ungesättigte Alkohole wie Allylalkohol, Trimethylolpropandiallylether, Butendiol und monofunktionelle Alkohole, die sich von entsprechenden Säuren bzw. Säuregemischen aus ungesättigten synthetischen und natürlichen Fettsäuren ableiten.

Grundsätzlich möglich, jedoch keinesfalls bevorzugt, ist auch die (Mit)Verwendung von Ethergruppen aufweisenden Alkoxylierungsprodukten der oben beispielhaft genannten ein- und mehrwertigen Alkohole und/oder die (Mit)Verwendung von Hydroxylgruppen aufweisenden Umesterungsprodukten von Fetten oder Ölen mit mehrwertigen Alkoholen wie insbesondere Glycerin, Trimethylolpropan, Pentaerythrit.

Das Molekulargewicht der Komponente a) wird durch geeignete Auswahl der Ausgangskomponenten a1) und a2) und insbesondere durch deren mittlere Funktionalität eingestellt. Da als Komponente a) hochmolekulare Verbindungen nicht in Betracht kommen, müssen dementsprechend als Komponente a 1) und/oder als Komponente a2) monofunktionelle Komponenten (mit)verwendet werden, um einen Kettenabbruch während der Urethanbildungsreaktion zu bewirken. Dies bedeutet, daß die mittlere Funktionalität der Komponenten a1) und a2) unter 2 liegt.

Die Isocyanatkomponente b) besteht aus destillierbaren organischen Polyisocyanaten der bereits oben bei der Beschreibung der Komponente a1) genannten Art mit Ausnahme von 2,2'-, 2,4'- und 4,4'-Diisocyanatodiphenylmethan (s. auch US-A-5319054), wobei jedoch die Komponente b) keine Monoisocyanate aufweisen sollte. Die (mittlere) NCO-Funktionalität der Komponente b) liegt bei 2 bis 4, vorzugsweise bei 2, d.h. es werden vorzugsweise ausschließlich Diisocyanate als Komponente b) eingesetzt.

Besonders bevorzugt werden als Komponente b) TDI, IPDI, HTDI , HMDI oder HDI eingesetzt.

Der erfindungswesentliche Punkt besteht nun darin, daß als Komponente a1) einerseits und als Komponente b) andererseits unterschiedliche Isocyanate der beispielhaft genannten Art Verwendung finden. Dies bedeutet, daß bei Verwendung von Isocyanatgemischen als Komponente a1) und/oder Komponente b) maximal 10 Gew.-% der in den jeweiligen Komponenten vorliegenden Isocyanate die gleichen sind. Vorzugsweise besteht selbstverständlich die Komponente b) ausschließlich aus solchen Isocyanaten, die in der Komponente a1) nicht vorliegen. Hierbei gelten Stellungsisomere Isocyanate gleichen Typs, wie beispielsweise 2,4-und 2,6-TDI, 2,4- und 2,6-HTDI, 2,4'-, 2,2'- und 4,4'-MDI, 2,4'-, 2,2'- und 4,4'-HMDI nicht als unterschiedliche Isocyanate.

Besonders bevorzugt sind Kombinationen bestehend aus
- HDI als Komponente a1) und TDI als Komponente b),
- HDI als Komponente a1) und IPDI als Komponente b),
- HDI als Komponente a1) und HTDI als Komponente b),
- HMDI als Komponente a1) und HDI als Komponente b),
- IPDI als Komponente a1) und HDI als Komponente b)
   und
- HTDI als Komponente a1) und HDI als Komponente b).

Die Umsetzung der Urethankomponente a) mit der Isocyanatkomponente b) erfolgt in einem NCO/Urethan-Äquivalentverhältnis von 3:1 bis 100:1 vorzugsweise von 6:1 bis 60:1 und besonders bevorzugt von 8:1 bis 30:1 im Temperaturbereich von 40°C bis 150°C, vorzugsweise 50°C bis 120°C und besonders bevorzugt 60°C bis 90°C, wobei vorzugsweise zur Beschleunigung der Allophanatisierungsreaktion übliche, Literatur bekannte Katalysatoren eingesetzt werden. Beispiele für einsetzbare Katalysatoren sind Tetraalkylammoniumhydroxide bzw. Arylalkylammoniumhydroxide, Metallsalze wie Eisen-(III)-chlorid, Kaliumoctoat, Zinkverbindungen wie Zinkstearat, Zinkoctoat, Zinknaphthenat, Zinkacetylacetonat, Zinnverbindungen wie Zinn-(II)-octoat, Zinn-(II)-ethylhexanoat, Zinn-(II)-laurat, Aluminium-tri(ethlylacetoacetat), Dibutylzinnoxid, Dibutylzinndichlorid, Dibutylzinndiacetat, Dibutylzinndilaurat, Dibutylzimimaleat oder Dioctylzinndiacetat, Mangan-, Cobalt- und Nickelverbindungen sowie Mineralsäuren wie Trifluoressigsäure, Schwefelsäure, Chlorwasserstoff, Bromwasserstoff, Phosphorsäure oder Perchlorsäure.

Die Katalysatoren können vor der Allophanatisierungsreaktion oder auch schon vor der Urethanisierung zugesetzt werden. Sie kommen in Konzentrationen von 0,001 bis 5 Gew.-%, vorzugsweise 0,005 bis 1 Gew.-% zum Einsatz. Der Katalysator kann, wenn möglich, destillativ aus dem Reaktionsansatz entfernt werden. Es kann jedoch auch sinnvoll sein, durch geeignete Katalysatorgifte seine katalytische Wirkung zu stoppen.

Durch geeignete Wahl der Ausgangskomponenten a) und b) wird die Funktionalität der entstehenden Verfahrensprodukte eingestellt. Die Allophanatisierungsreaktion wird bei Erreichen der angestrebten NCO-Funktionalität der Umsetzungsprodukte beendet. Im Anschluß an die Umsetzung wird der Überschuß an nicht umgesetzter Ausgangskomponente b), vorzugsweise durch Dünnschichtdestillation, bis auf einen Restgehalt im Verfahrensprodukt unter 0,5, vorzugsweise unter 0,2 Gew.-% entfernt.

Die erfindungsgemäßen Verfahrensprodukte stellen Allophanatgruppen aufweisende Polyisocyanate mit einem NCO-Gehalt von 5 bis 17, vorzugsweise 6 bis 15 Gew.-% und einem Gehalt an destillierbaren Isocyanaten von unter 0,5 und vorzugsweise unter 0,2 Gew.-% dar. Es handelt sich uni viskose bis harzartige Produkte.

Je nach Viskosität der erfindungsgemäßen Verfahrensprodukte kann es sinnvoll sein, diese mit inerten Lösungsmittel zu verdünnen. Hierfür geeignete Lösungsmittel sind beispielsweise Toluol, Xylol, Cyclohexan, Chlorbenzol, Butylacetat, Ethylacetat, Ethylglykolacetat, Pentylacetat, Hexylacetat, Methoxypropylacetat, Tetrahydrofuran, Dioxan, Aceton, Methylethylketon, Testbenzin, höher substituierte Aromaten, wie beispielsweise unter der Bezeichnung Solvent Naphtha®, Solvesso®, Shellsol®, Isopar®, Nappar® und Diasol® im Handel sowie Gemische derartiger Lösungsmittel.

Die erfindungsgemäßen Verfahrensprodukte stellen unter dem Einfluß von Luftfeuchtigkeit aushärtbare Bindemittel für Einkomponenten-Beschichtungsmaterialien dar. Vorzugsweise werden die erfindungsgemäßen Verfahrensprodukte jedoch als Vernetzer in Zweikomponenten-Polyurethanlacken eingesetzt. Grundsätzlich möglich ist auch die Verwendung der erfindungsgemäßen Verfahrensprodukte in mit an sich bekannten Blockierungsmitteln blockierter Form in Kombination mit an sich bekannten Polyhydroxylverbindungen in thermohärtenden Einkomponenten-Beschichtungssystemen.

Selbstverständlich können in allen Beschichtungsmitteln, in denen die erfindungsgemäßen Verfahrensprodukte als Bindemittel bzw. als Bindemittelkomponente eingesetzt werden, die aus der Lacktechnologie an sich bekannten Hilfs- und Zusatzmittel mitverwendet werden. In diesem Zusammenhang beispielhaft genannt seien Benetzungsmittel, Verlaufsmittel, Hautverhinderungsmittel, Antischaummittel, Mattierungsmittel wie beispielsweise Kieselsäure, Aluminiumsilikate und hochsiedende Wachse, viskositätsregulierende Stoffe, Pigmente, Farbstoffe, UV-Absorber oder Stabilisatoren gegen thermischen bzw. oxidativen Abbau.

Die die erfindungsgemäßen Verfahrensprodukte als Bindemittel oder Bindemittelkomponente enthaltenden Beschichtungsmittel können zur Beschichtung beliebiger Substrate wie beispielsweise Holz, Kunststoffe, Leder, Papier, Textilien, Glas, Keramik, Putz, Mauerwerk, Metalle oder Beton verwendet werden. Sie lassen sich mit üblichen Applikationsmethoden wie Spritzen, Streichen, Fluten, Gießen, Tauchen, Walzen aufbringen.

Vor- und auch nachstehend beziehen sich alle Viskositätsangaben auf die bei 23°C rotationsviskosimetrisch gemäß DIN 53 019 bestimmten Viskositäten.

In den nachfolgenden Beispielen beziehen sich alle Angaben in "%" auf das Gewicht.

### Beispiel 1

In einer mit Stickstoff gespülten Rührapparatur werden 29,5 g (0,226 Mol) 2-Ethylhexanol vorgelegt und bei 60°C mit 19 g (0,113 Mol) HDI versetzt. Nach ca. 6 Stunden Reaktionsdauer bei einer Temperatur von 95°C ist der NCO-Gehalt des entstandenen Diurethans auf unter 0,2 % gefallen. Nachfolgend wird durch Zugabe von 236 g TDI (2,4- und 2,6-Diisocyanatotoluol im Gewichtsverhältnis 80:20) und anschließende Katalyse mit 43 mg Zinkstearat bei 88°C die Allophanatisierungsreaktion gestartet. Nach 6 Stunden wird noch einmal mit 14 mg Katalysator nachaktiviert und weitergerührt bis ein konstanter NCO-Gehalt von 36,2 % erreicht ist. Das überschüssige TDI wird im Anschluß durch Dünnschichtdestillation im Hochvakuum (0,1 - 0,3 mbar) bei einer Temperatur von 150 °C abgetrennt.

### Produktdaten:

- Ausbeute:: 83 g
- NCO-Gehalt:: 10,8 %
- Viskosität:: 75 000 mPas/23°C
- fr. TDI-Gehalt:: < 0,03 %

Das ¹³C-NMR-Spektrum zeigt die für Allophanate charakteristischen Peaks jedoch keine Signale für Urethangruppen. Auch der niedrige Gehalt an freiem TDI weist auf eine einfache Abtrennung des Monomers während der Destillation ohne Spaltung hin.

### Beispiel 2

In einer mit Stickstoff gespülten Rührapparatur werden 29,5 g (0,226 Mol) 2-Ethylhexanol vorgelegt und bei 60°C mit 19 g (0,113 Mol) HDI versetzt. Nach ca. 6 Stunden Reaktionsdauer bei einer Temperatur von 95°C ist der NCO-Gehalt des entstandenen Diurethans auf unter 0,2 % gefallen. Nachfolgend wird durch Zugabe von 302 g (1,36 Mol) IPDI und anschließende Katalyse mit 0,35 g Zinkstearat bei 88°C die Allophanatisierungsreaktion gestartet. Nach 6 Stunden ist ein konstanter NCO-Gehalt von 29,83 % erreicht. Das überschüssige IPDI wird im Anschluß durch Dünnschichtdestillation im Hochvakuum (0,1 bis 0,3 mbar) bei einer Temperatur von 150°C abgetrennt.

### Produktdaten:

- Ausbeute:: 90 g
- NCO-Gehalt:: 9,2 %
- Viskosität:: 85 000 mPas/23°C
- fr. IPDI-Gehalt:: 0,15 %

## Patentansprüche

1. Verfahren zur Herstellung von Allophanatgruppen aufweisenden Polyisocyanaten durch Umsetzung unter Allophanatbildung von Urethangruppen aufweisenden Verbindungen a) mit bezüglich der Urethangruppen überschüssigen Mengen an destillierbaren organischen Polyisocyanaten b) und anschließende destillative Entfernung des nicht umgesetzten Überschusses der Komponente b) bis auf einen Restgehalt von unter 0,5 Gew.-%, und wobei die
als Komponenten a) im wesentlichen Hydroxyl- und Isocyanatgruppen-freie, pro Molekül im statistischen Mittel mindestens zwei Urethangruppen aufweisende, durch Umsetzung von organischen Isocyanaten a1) mit organischen Hydroxylverbindungen a2) hergestellte Verbindungen eines maximalen durchschnittlichen Molekulargewichts von 2500 sind dadurch gekennzeichnet, daß man
als Komponente b) von der Komponente a1) verschiedene Polyisocyanate verwendet, wobei 2,2'-, 2,4'- und 4,4'-Diisocyanatodiphenylmethan ausgenommen sind,

2. Verfahren nach Anspruch 1, dadurch gekennzeichnet, daß man als Komponente a1) Diisocyanate, ausgewählt aus der Gruppe bestehend aus (i) Hexamethylendiisocyanat, (ii) 4,4'-Diisocyanato-dicyclohexylmethan oder dessen Gemischen mit seinen 2,4'- und gegebenenfalls 2,2'-Isomeren, (iii) 1-Isocyanato-3,3,5-isocyanatomethyl-cyclohexan und (iv) 1-Methyl-2,4-diisocyanato-cyclohexan oder dessen Gemischen mit 1-Methyl-2,6-di-isocyanato-cyclohexan, verwendet.

3. Verfahren gemäß Anspruch 1 und 2, dadurch gekennzeichnet, daß man als Komponente a2) 1- bis 6-wertige Alkohole des Molekulargewichtsbereichs 32 bis 900 oder Gemische derartiger Alkohole verwendet.

4. Verfahren gemäß Anspruch 1 bis 3, dadurch gekennzeichnet, daß man als Komponente b) organische Diisocyanate, ausgewählt aus der Gruppe bestehend aus (i) 2,4-Diisocyanatotoluol oder dessen Gemischen mit 2,6-Diisocyanatotoluol, (ii) Hexamethylendiisocyanat, (iii) 1-Isocyanato-3,3,5-trimethyl-5-isocyanatomethyl und (iv) 1-Methyl-2,4-diisocyanato-cyclohexan oder dessen Gemischen mit 1-Methyl-2,6-diisocyanato-cyclohexan, verwendet.

5. Verfahren gemäß Anspruch 1 bis 4, dadurch gekennzeichnet, daß man als Komponente a1) Hexamethylendiisocyanat und als Komponente b) (i) 2,4-Diisocyanatotoluol oder dessen technischen Gemische mit 2,6-Diiso-cyanatotoluol oder (ii) 1-Isocyanato-3,3,5-trimethyl-5-isocyanatomethyl-cyclohexan verwendet.

6. Verfahren gemäß Anspruch 1 bis 4, dadurch gekennzeichnet, daß man als Komponente a1) 4,4'-Diisocyanato-dicyclohexylmethan oder dessen Gemische mit seinen 2,4'- und gegebenenfalls 2,2'-Isomeren und als Komponente b) Hexamethylendiisocyanat verwendet.

7. Gemäß Anspruch 1 bis 6 erhältliche, Allophanatgruppen aufweisende Polyisocyanate.

8. Verwendung der nach Anspruch 1 bis 7 erhältlichen Polyisocyanate als Bindemittel oder Bindemittelkomponente in Beschichtungsmitteln.

## Claims

1. Method for preparing polyisocyanates possessing allophanate groups by reacting compounds a) possessing urethane groups with excess quantities relative to the urethane groups of distillable organic polyisocyanates b), with the formation of allophanate, and subsequent removal by distillation of the unreacted excess of component b) to a residual content of less than 0.5 wt.%, and wherein
components a) are compounds which are substantially free of hydroxyl groups and isocyanate groups, have on statistical average at least two urethane groups per molecule, are prepared by reacting organic isocyanates a1) with organic hydroxyl compounds a2) and have an average molecular weight of 2,500 at most,
characterised in that polyisocyanates different from component a1) are used as component b), 2,2'-, 2,4'-and 4,4'-diisocyanatodiphenylmethane being excluded.

2. Method according to claim 1, characterised in that diisocyanates selected from the group comprising (i) hexamethylene diisocyanate, (ii) 4,4'-diisocyanato-dicyclohexylmethane or mixtures thereof with its 2,4'-and optionally 2,2'-isomers, (iii) 1-isocyanato-3,3,5-isocyanatomethylcyclohexane and (iv) 1-methyl-2,4-diisocyanatocyclohexane or mixtures thereof with 1-methyl-2,6-diisocyanatocyclohexane are used as component a1).

3. Method according to claims 1 and 2, characterised in that monohydric to hexahydric alcohols in the molecular weight range of 32 to 900 or mixtures of such alcohols are used as component a2).

4. Method according to claims 1 to 3, characterised in that organic diisocyanates selected from the group comprising (i) 2,4-diisocyanatotoluene or mixtures thereof with 2,6-diisocyanatotoluene, (ii) hexamethylene diisocyanate, (iii) 1-isocyanato-3,3,5-trimethyl-5-isocyanatomethylcyclohexane and (iv) 1-methyl-2,4-diisocyanatocyclohexane or mixtures thereof with 1-methyl-2,6-diisocyanatocyclohexane are used as component b).

5. Method according to claims 1 to 4, characterised in that hexamethylene diisocyanate is used as component a1) and (i) 2,4-diisocyanatotoluene or technical mixtures thereof with 2,6-diisocyanatotoluene or (ii) 1-isocyanato-3,3,5-trimethyl-5-isocyanatomethyl-cyclohexane are used as component b).

6. Method according to claims 1 to 4, characterised in that 4,4'-diisocyanatodicyclohexylmethane or mixtures thereof with its 2,4'- and optionally 2,2'-isomers are used as component a1) and hexamethylene diisocyanate is used as component b).

7. Polyisocyanates possessing allophanate groups obtainable according to claims 1 to 6.

8. Use of the polyisocyanates obtainable according to claims 1 to 7 as binders or binder components in coating materials.

## Revendications

1. Procédé pour la préparation de polyisocyanates présentant des groupes allophanate par réaction avec formation d'allophanate de composés présentant des groupes uréthane a) avec des quantités en excès par rapport aux groupes uréthane de polyisocyanates organiques pouvant être distillés b) et par élimination subséquente par distillation de l'excès du constituant b) n'ayant pas réagi jusqu'à une teneur résiduelle inférieure à 0,5 % en poids, et les constituants a) étant des composés en principe exempts de groupes hydroxyle et isocyanate, présentant en moyenne statistique par molécule au moins deux groupes uréthane, préparés par réaction d'isocyanates organiques a1) avec des composés hydroxyle organiques a2) d'un poids moléculaire moyen maximal de 2 500, caractérisé en ce que l'on utilise comme constituant b) des polyisocyanates différents des constituants a1), le 2,2'-, 2,4'- et 4,4'-diisocyanatodiphénylméthane étant exclus.

2. Procédé selon la revendication 1, caractérisé en ce que l'on utilise comme constituant a1) des diisocyanates choisis parmi (i) le diisocyanate d'hexaméthylène, (ii) le 4,4'-diisocyanato-dicyclohexylméthane ou des mélanges de celui-ci avec ses isomères 2,4' et éventuellement 2,2', (iii) le 1-isocyanato-3,3,5-isocyanatométhyl-cyclohexane et (iv) le 1-méthyl-2,4-diisocyanato-cyclohexane ou des mélanges de celui-ci avec du 1 méthyl-2,6-diisocyanato-cyclohexane.

3. Procédé selon la revendication 1 ou 2, caractérisé en ce que l'on utilise comme constituant a2) des alcools mono-à hexavalents du domaine de poids moléculaire 32 à 900 ou des mélanges d'alcools de ce type.

4. Procédé selon la revendication 1 à 3, caractérisé en ce que l'on utilise comme constituant b) des diisocyanates organiques choisis parmi (i) le 2,4-diisocyanatotoluène ou des mélanges de celui-ci avec du 2,6-diisocyanatotoluène, (ii) le diisocyanate d'hexaméthylène, (iii) le 1-isocyanato-3,3,5-triméthyl-5-isocyanatométhyle et (iv) le 1-méthyl-2,4-diisocyanato-cyclohexane ou des mélanges de celui-ci avec du 1-méthyl-2,6-diisocyanato-cyclohexane.

5. Procédé selon la revendication 1 à 4, caractérisé en ce que l'on utilise comme constituant a1) du diisocyanate d'hexaméthylène et comme constituant b) (i) du 2,4-diisocyanatotoluène ou des mélanges industriels de celui-ci avec du 2,6-diisocyanatotoluène ou (ii) du 1-isocyanato-3,3,5-triméthyl-5-isocyanatométhylcyclohexane.

6. Procédé selon la revendication 1 à 4, caractérisé en ce que l'on utilise comme constituant a1) du 4,4'-diisocyanato-dicyclohexylméthane ou des mélanges de celui-ci avec ses isomères 2,4' et éventuellement 2,2' et comme constituant b) le diisocyanate d'hexaméthylène.

7. Polyisocyanates présentant des groupes allophanate obtenus selon l'une quelconque des revendications 1 à 6.

8. Utilisation des polyisocyanates obtenus selon l'une quelconque des revendications 1 à 7 comme liants ou constituants de liants dans des agents de revêtement.
